(19) Europäisches Patentamt
European Patent Office
Office européen des brevets

(11) Publication number: **0 214 709 B1**

(12) # EUROPEAN PATENT SPECIFICATION

(45) Date of publication of patent specification: **29.09.93**

(51) Int. Cl.[5]: **C07K 7/00**, A61K 37/02, C07K 15/00, C12P 21/00, G01N 33/569, //A61K39/21

(21) Application number: **86303224.9**

(22) Date of filing: **29.04.86**

The file contains technical information submitted after the application was filed and not included in this specification

(54) **Synthetic peptide and process of using same for the detection of antibodies to HTLV-III, diagnosis of AIDS and pre-AIDS conditions and as a vaccine.**

(30) Priority: **11.09.85 US 774644**
**04.03.86 US 837566**
**02.04.86 US 847102**

(43) Date of publication of application:
**18.03.87 Bulletin 87/12**

(45) Publication of the grant of the patent:
**29.09.93 Bulletin 93/39**

(84) Designated Contracting States:
**AT BE CH DE FR GB IT LI LU NL SE**

(56) References cited:
**EP-A- 0 044 710**
**EP-A- 0 201 716**
**WO-A-86/01834**
**WO-A-86/06414**

**CHEMICAL ABSTRACTS, vol. 107, 1987, page 590, no. 234358c, Columbus, Ohio, US; J.W. GNANN Jr. et al.: "Diagnosis of AIDS by using a 12-amino acid peptide representing an immunodominant epitope of the human immunodeficiency virus"**

**PROC. NATL. ACAD. SCI. USA, vol. 83, August 1986, pages 6159-6163; J.J.G. WANG et al.: "Detection of antibodies to human T-lymphotropic virus type III by using a synthetic peptide of 21 amino acid residues corresponding to a highly antigenic segment of gp41 envelope protein" (description of the invention)**

(73) Proprietor: **United Biomedical Inc.**
**2 Nevada Drive**
**Lake Success New York 11042(US)**

(72) Inventor: **Wang, James Jian Guang**
**137-27 Holly Avenue**
**Flushing New York 11355(US)**
Inventor: **Wang, Chang Yi**
**77 Park Avenue**
**New York New York 10016(US)**

(74) Representative: **Froud, Clive et al**
**Elkington and Fife Prospect House 8 Pembroke Road**
**Sevenoaks, Kent TN13 1XR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention relates to a synthetic peptide and process of using same for the detection of antibodies to HTLV-III (AIDS virus, later termed HIV), diagnosis of AIDS and pre-AIDS conditions and as a vaccine.

In one embodiment the present invention provides a peptide composition having specific immunoreactivity to antibodies to the AIDS virus characterised in that it is selected from peptides containing from twelve to twenty-one amino acids, the sequence of which corresponds to a part or the entirety of the following:

Arg-Ile-Leu-Ala-Val-Glu-Arg-Tyr-Leu-Lys-Asp-Gln-Gln-Leu-Leu-Gly-Ile-Trp-Gly-Cys-Ser;

analogues thereof wherein the amino acids in the sequence are substituted, deleted or added as long as the immunoreactivity to antibodies to the AIDS virus is preserved; and conjugates of the peptides or analogues thereof.

More particularly, the present invention relates to a highly sensitive method for the detection of antibodies to HTLV-III in body fluids and diagnosis of AIDS (acquired immune deficiency syndrome), ARC (AIDS Related Complex) and pre-AIDS conditions by the use of a chemically synthesized peptide, a vaccine for AIDS, ARC or pre-AIDS conditions and a method for stimulating the production of antibodies to HTLV-III to provide protection against infection by HTLV-III or LAV in healthy mammals, including humans. The amino acid sequence of the peptide corresponds to a segment of the envelope protein, p41, of HTLV-III and has been found to be highly immunoreactive with antibodies in sera of patients with AIDS, ARC and pre-AIDS conditions. More specifically, the present invention is directed to the use of a chemically synthesized peptide containing therein a segment of about twenty-one amino acids, or their analogues, in a prescribed sequence for the detection of antibodies to the HTLV-III virus in human body fluids of AIDS, ARC or pre-AIDS patients. The detection method includes an enzyme-linked immunosorbent assay (ELISA) and an immunoradiometric assay (IRMA) and other methods of immuno assay procedures, such as enzyme immunoblotting on nitrocellulose paper and hemagglutination using the peptide as the antigen. The preferred detection method is ELISA.

Acquired immune deficiency syndrome (AIDS) has been recently recognized in several countries. Due to its devastating effect on the patients and indications that the disease is spreading, much effort has been devoted to elucidate and identify the cause of the disease. Epidemiologic data suggest that AIDS is caused by an infectious agent that is horizontally transmitted by intimate contact or exposure to blood or certain blood products.

In 1983, F. Barre-Sinoussi et al. of the Institute Pasteur reported the isolation of a T-lymphotropic retrovirus from a patient at risk for AIDS. The retrovirus appeared to be a member of the human T-cell leukemia virus (HTLV) family. However, its immunological response is distinct from known HTLV-I or HTLV-II. (F. Barre-Sinoussi et al., Science, 220, pp. 868, May, 1983).

A similar virus, designated HTLV-III, has also been isolated by R.C. Gallo's group at the National Cancer Institute, U.S.A. from the blood samples of a large number of AIDS and ARC patients by co-cultivation with a permissive T-cell line H9. (See Popovic, M. et al., Science, 224, pp. 497, 1984, and Gallo, R., et al., Science, 224, pp. 500, 1984).

V.S. Kalyanaraman et al. of the Center for Disease Control, Atlanta, Georgia, U.S.A., reported the isolation of a lymphadenopathy associated virus (LAV) in patients with AIDS and the development of a radioimmuno-precipitation assay using the major core protein, p25, of LAV. Their test procedure involved the use of the LAV virus propagated in primary cultures of blood lymphocytes and harvested. The p25 core protein was isolated from the harvested virus, labelled with $I^{125}$ and used as target antigen. The labelled antigen was added to serum and precipitation of at least 15% of the labelled antigen is regarded as a positive result. (See V.S. Kalyanaraman et al., Science, 225, pp. 321, July, 1984). However, based on the reported results, the test was positive only for 41% of the AIDS patients and 72% positive for patients with lymphadenopathy syndrome (LAS), otherwise known as ARC. This means that the procedure is not sufficiently sensitive or accurate to be used as a detection or diagnostic tool for screening serum for the presence of antibodies to the AIDS virus.

LAV and HTLV-III, as well as various strains of related retrovirus isolated from AIDS patients share several important characteristics. (See Feorino, P., et al., Science, 225, pp. 69, 1984; Levy, J., et al., Science, 225, pp. 840, 1984). These include viral replication in $OKT4^+$ human T-cell leukocytes, in vivo and in vitro; association with impaired T-cell proliferation, the appearance of cytopathic effects; (See Montagnier et al., Human T Cell Leukemia Virus, pp. 363, Cold Spring Harbor Laboratory, 1984; Popovic, M., et al., op.

cit.; and Klatzmann, D., et al. Science, 225, pp. 59, 1984) and recognition by antibodies in the sera of AIDS and ARC patients. (See Montagnier, et al., op. cit.; Levy J. et al op. cit. Sarngadharan M. et al., Science, 224, pp. 505, 1984; Safai, B., et al., Lancet, i. pp. 1438, 1984; Brun-Vezinet, F., et al., Lancet, i, pp. 1253, 1984; Brun-Vezinet, F., et al., Science, 226, pp. 453, 1984; Goldbert, J., et al., Lancet, ii, pp. 711, 1984, and Laurence, J., et al., New England J. Med., 311, pp. 1269, Nov. 1984.

In November 1984, L.W. Kitchen et al. reported the use of a HTLV-III infected line, designated H9/HTLV-III, to test the incidents of AIDS in haemophiliac patients. The method involved inactivation of the virus with 2% paraformaldehyde in phosphate buffer and use of the unactivated cells to determine if haemophiliac patients have been inadvertently exposed to AIDS virus through blood transfusion. The data using sera samples from 50 haemophiliacs show that there is an increasing risk for these patients to contract AIDS, because of their need for blood tranfusions to sustain life. (L.W. Kitchen, et al., Nature, 312, pp. 367 Nov., 1984). This means that there is an urgent need for a safe, reliable and sensitive test to screen each blood sample before its inclusion in blood banks to isolate blood samples which have been contaminated with AIDS virus, and thus avoid the inadvertent spread of AIDS among patients who must receive blood transfusions, e.g. haemophiliac and surgical patients.

In November 1984 and January 1985, R.C. Gallo's group at the National Cancer Institute, U.S.A., and other collaborators positively concluded that HTLV-III is the causative agent of AIDS and reported the nucleotide sequence of HTLV-III. (See Beatrice Hahn, et al., nature, 312, pp. 166, Nov., 1984; George M. Shaw, et al., Science, 226, pp. 1165, Dec. 1984; and Lee Ratner et al., Nature, 313, pp. 277, Jan., 1985).

Meanwhile, three other groups also reported the complete nucleotide sequence of the AIDS virus. (See Muesing et al., Nature, 313, pp. 450, Feb., 1985; Sanchez-Pescados, R., et al., Science, 227, pp. 484, Feb., 1985; and Wain-Hobson et al., Cell, 40, pp. 9, Jan., 1985). These reports elucidated the structure of the HTLV-III virus at both the DNA level and the projected protein level and permit further serological studies of the different epitopes present on the HTLV-III virus.

Simultaneously, the group at Institute Pasteur reported that LAV has been identified as a causative agent for AIDS, and is considered to be identical to HTLV-III. The assay procedure used by this group also involves propagating LAV in $T4^+$ cells of healthy individuals. The viral antigen was then concentrated and deactivated in 0.5 per cent sodium dodecyl sulfate at 37°C for 15 minutes. Serum samples were then tested against the antigen in an enzyme immunoassay with orthophenylene diamine as substrate. The presence of antibody in serum was found in 68% of AIDS patients, 100% of patients with Kaposi's sarcoma and 100% of pre-AIDS patients. (Jeffrey Laurence et al., op. cit.)

Recently, U.S. Patent No. 4,520,113 was issued to R.C. Gallo et al. This patent describes a method of detecting antibodies in sera of AIDS and pre-AIDS patients by using lysates of a cell line, designated H9/HTLV-III, as the antigen in an enzyme-linked immunosorbent assay (ELISA) or in a strip radioimmunoassay based on the Western Blot technique or an indirect immunofluorescent method. The method is about 85% accurate. The Gallo patent further indicated that several antigens from HTLV-III, p65, (MW 65,000), p60 (MW 60,000), p55 (MW 55,000), p24 (MW 24,000) and p41 (MW 41,000) are recognized by antibodies in sera from AIDS patients, homosexuals and heroin addicts. Of these, major immune reactivity or specificity is directed against p41, a protein constituting the envelope antigen of HTLV-III. This patent further states that it is believed that additional purification and refinement of p41 may lead to an even more sensitive ELISA assay. Based on this statement, the antigen suitable as a test reagent is to be a p41 segment derived from HTLV-III cultivated in H9 cell line.

It is further reported in Robert C. Gallo et al., Science, 228, pp. 93, April, 1985, that a combined cloning and expression system in E. Coli has been used to identify HTLV-III encoded peptides which react immunologically with antibodies in sera from AIDS patients. Cloned HTLV-III DNA was sheared into fragments and inserted into an expression vector. The inserted DNA was then expressed in E. Coli transformants. Of 300 clones tested, 20 showed specific reactivity with sera from AIDS patients. The 20 clones were analyzed and found to contain segments from the ORF segment of HTLV-III and were identified as clones 175, 191, 13, 31, 162, 113, 121 and 127. Of the eight clones, ORF clones 113, 121 and 127 define the protein encoded by the portion of the env-lor region produced by HTLV-III infected cells and induces antibody production in most if not all AIDS patients.

Also, EP-A-201716 discloses recombinantly-produced immunoreactive LAV envelope proteins and WO86/06414 relates to synthetic antigen for the detection of AIDS - related diseases.

All of the reported assay procedures for detecting antibodies to HTLV-III and for diagnosis of AIDS or preAIDs conditions involve the use of the HTLV-III or LAV virus. None of the procedures are 100% accurate. This is undesirable for use in the screening of sera in blood banks. The less than 100% accuracy of the tests may permit contaminated sera from escaping detection and be used in blood transfusions to the severe detriment of blood recipients. Moreover, the use of the HTLV-III virus as the testing agent is

dangerous to healthy laboratory workers, requiring extreme precautions to avoid all chances of exposure during the preparative process to make the test reagent. Furthermore, even though the deactivated virus is used in some of the published procedures, exposure to the deactivated virus can cause antibody production in healthy workers, who may then be falsely diagnosed as having AIDS, ARC or pre-AIDS condition. Moreover, presence of cellular materials from H9 cells or E. Coli in the test agent may elicit a false positive response in the HTLV-III antibodies screening test from individuals who have antibodies to E. Coli or H9 cells. These false positive reactions can bring severe anxiety to the healthy individuals and their family and may lead to a healthy individual being mistakenly diagnosed as having AIDS and be exiled from normal social activities as a consequence.

Furthermore, up to the present, no viable vaccine or method to provide protection against HTLV-III has been reported for AIDS, ARC or pre-AIDS conditions. The use of deactivated virus provokes fears of contracting the disease and would prevent its acceptability and use.

Similarly, the development of monoclonal and polyclonal antibodies to HTLV-III in mammals involves the use of HTLV-III as the immunogen and this presents similar risks in the procedure.

It is, therefore, an object of the present invention to develop a detection or diagnostic procedure that does not require the use of the virus or lysates thereof as a test reagent.

A further object is to develop a test procedure that is highly sensitive and accurate, preferably 100% accurate.

Another object is to develop a test that is highly sensitive so that very little test reagent or body fluid is needed to obtain an accurate result.

Still another object is to prepare a test reagent by chemical means, which test reagent can be used to detect the presence of antibodies to HTLV-III in body fluids and diagnose AIDS, ARC or pre-AIDS conditions, thereby avoiding the danger of exposure to the virus or segments thereof and the unnecessary proliferation of the virus.

Yet another object is to develop a vaccine which when introduced into the body will stimulate production of antibodies to HTLV-III to provide protection against infection by HTLV-III or LAV in healthy mammals, including humans.

A further object is to provide an immunogen which can be used for the development in mammals of monoclonal and polyclonal antibodies to HTLV-III which does not involve the use of HTLV-III as the immunogen.

According to the present invention, a peptide with about twenty one amino acids arranged in a specific sequence has been made by solid phase peptide synthesis. The 21mer peptide has been found to be useful in a highly sensitive and accurate method for the detection of antibodies to HTLV-III in sera and body fluids and diagnosis of AIDS, ARC or pre-AIDS conditions. The 21mer peptide has also been found to be useful in stimulating production of antibodies to HTLV-III or LAV in healthy mammals, such as Balb/c mice.

According to the present invention, a peptide useful for the detection of antibodies to HTLV-III and diagnosis of AIDS, ARC or pre-AIDS conditions in sera or body fluids is selected from peptides comprising:

**Arg-Ile-Leu-Ala-Val-Glu-Arg-Tyr-Leu-Lys-Asp-Gln-Gln-Leu-Leu-Gly-Ile-Trp-Gly-Cys-Ser,**

analogues thereof and segments thereof; wherein:

Ala = alanine,
Arg = arginine,
Asp = aspartic acid,
Gln = glutamine,
Glu = glutaminc acid,
Leu = Leucine,
Lys = Lysine,
Gly = glycine,
Ile = isoleucine,
Ser = serine,
Trp = tryptophan,
Tyr = tyrosine,
Val = valine, and
Cys = cysteine.

The highly sensitive and accurate method of detecting antibodies to HTLV-III in body fluids and diagnosis of AIDS, ARC or pre-AIDS conditions comprises:

(A) preparing a peptide selected from peptides comprising:

**Arg-Ile-Leu-Ala-Val-Glu-Arg-Tyr-Leu-Lys-Asp-Gln-Gln-Leu-Leu-Gly-Ile-Trp-Gly-Cys-Ser,**

analogues thereof and segments thereof:

(B) using from about 0.1 to about 20 µg per test of the peptide in a buffer at a pH of from about 7 to about 10 as the antigen in an immunoassay procedure.

Furthermore, according to the present invention, the peptide, when coupled to a protein carrier or a polymer carrier, can be used to stimulate production of antibodies to HTLV-III or LAV in healthy mammals including humans. The method comprises introducing an effective amount of the 21mer peptide conjugated to a protein, such as human serum albumin, into the body of a healthy mammal by interperitoneal or subcutaneous injection.

Referring to the accompanying illustrative drawing:

Fig. 1 is a graph comparing the data obtained with sera from AIDs patients using ELISA method wherein the well plates are coated with (A) the peptide of the present invention, (B) deactivated HTLV-III virus; and normal sera with (a) the peptide of the present invention (b) deactivated HTLV-III virus.

In accordance with the present invention, a peptide has been chemically synthesized for the detection of antibodies to HTLV-III in body fluids and diagnosis of AIDS, ARC and pre-AIDS conditions, for the vaccination of healthy mammals by stimulating the production of anitbodies to HTLV-III or LAV in healthy mammals, and for the development of both monoclonal and polyclonal antibodies to HTLV-III in mammals. The peptide comprise about twenty one amino acids or their analogues arranged in the following sequence:

Arg-Ile-Leu-Ala-Val-Glu-Arg-Tyr-Leu-Lys-Asp-Gln-Gln-Leu-Leu-Gly-Ile-Trp-Gly-Cys-Ser.

The peptide may comprise a shorter or longer peptide chain by having more amino acids added to the terminal amino acids, -Arg- or -Ser-, of the above sequence or having a few less of the terminal amino acids from either terminal.

It is expected that as long as the three dimensional conformation recognizable by the dominant antibodies to HTLV-III is preserved, the synthetic peptide may comprise analogues of the recited amino acids of the above sequence or be extended by the addition of amino acids to either terminal or it may be reduced by the deletion of a few of the terminal amino acids to at least about fifteen amino acids. In addition, it is expected that polymeric forms of the 21mer peptide or its analogues can also be used to elicit production of antibodies to HTLV-III.

The amino acid sequence of the polypeptide useful as a test reagent for the detection of antibodies to HTLV-III in body fluids and diagnosis of AIDS, ARC and pre-AIDS conditions is prepared to correspond to a partial segment of the amino acid sequence of the HTLV-III virus designated as p41, a part of p120, the LOR (long open reading) segment, defining the envelope protein of the HTLV-III virus.

The peptide useful as a solid phase immunoadsorbent for the detection of antibodies HTLV-III was synthesized by a "classical" Merrifield method of solid phase peptide synthesis according to the following scheme:

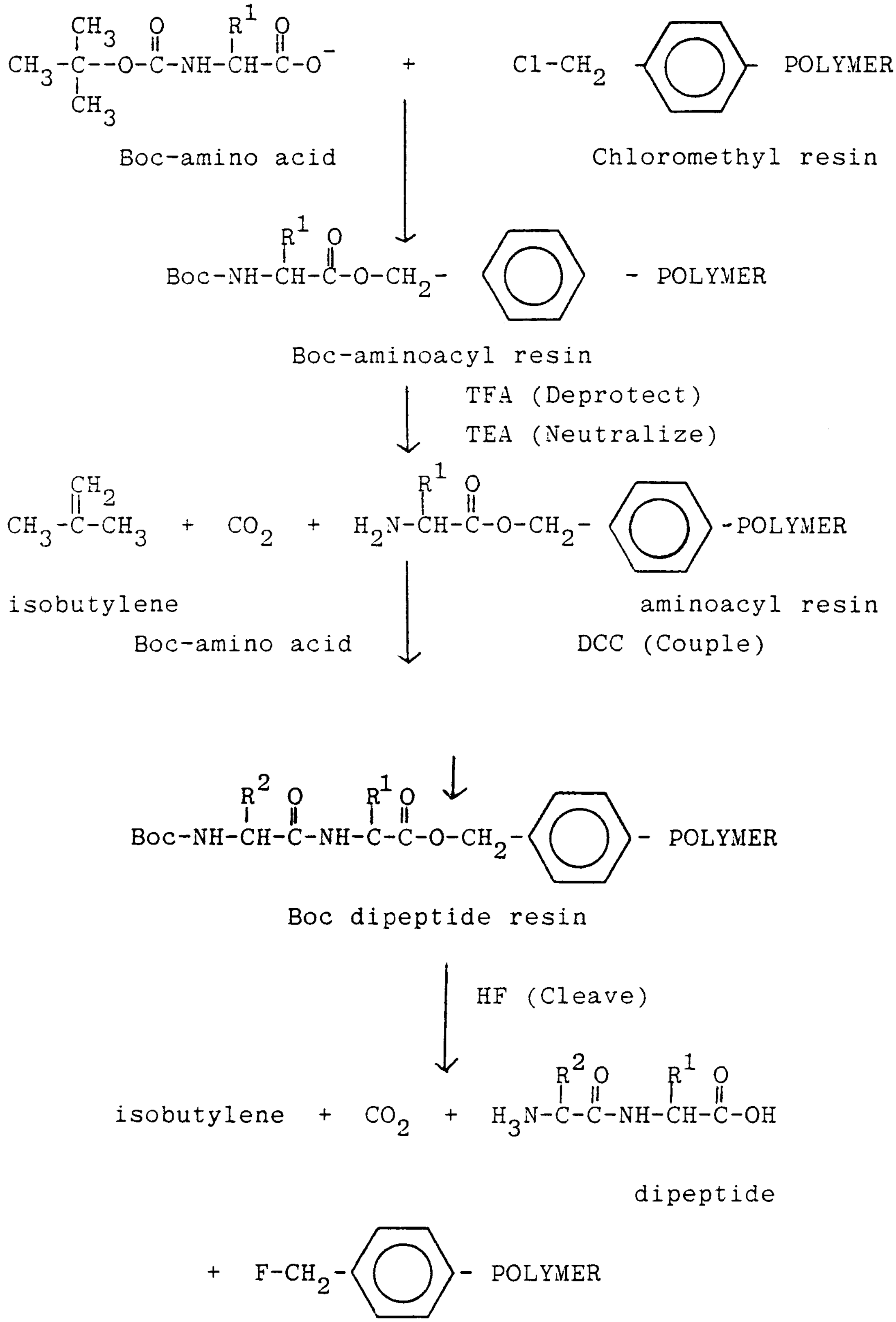


Following the above scheme, Boc-amino acids are added to the resin to prepare the 21mer peptide according to the following sequence:

| | | | | | |
|---|---|---|---|---|---|
| 1. | Boc-Ser | 8. | Boc-Leu | 15. | Boc-Arg |
| 2. | Boc-Cys | 9. | Boc-Gln | 16. | Boc-Glu |
| 3. | Boc-Gly | 10. | Boc-Gln | 17. | Boc-Val |
| 4. | Boc-Trp | 11. | Boc-Asp | 18. | Boc-Ala |
| 5. | Boc-Ile | 12. | Boc-Lys | 19. | Boc-Leu |
| 6. | Boc-Gly | 13. | Bcc-Leu | 20. | Boc-Ile |
| 7. | Boc-Leu | 14. | Boc-Tyr | 21. | Boc-Arg |

Analogues of the 21mer peptide can be prepared by varying the above sequence either by adding or subtracting desired Boc-amino acid(s). The term "analogues" of the 21mer peptide encompasses the entire 21mer peptide, portions thereof, peptides that contain substitutions of amino acid residues, or peptides that contain insertions and/or deletions of amino acids in the 21mer peptide.

Following completion of assembly of the desired blocked peptide on the resin, the peptide-resin is treated with anhydrous hydrofluoric acid to cleave the benzyl ester linking the peptide to the resin in order to liberate the peptide. Side-chain functional groups of amino acids which are blocked during synthesis by benzyl-derived blocking groups are also cleaved from the peptide simultaneously. The free peptide is then analyzed and purified by reverse phase high pressure liquid chromotography (HPLC) and characterized biochemically by amino acid analysis.

Similarly, synthesis of the 21mer peptide that has an amide group on its C-terminal end can be acheived by using a 4-methylbenzhydrylamine resin according to the following scheme:

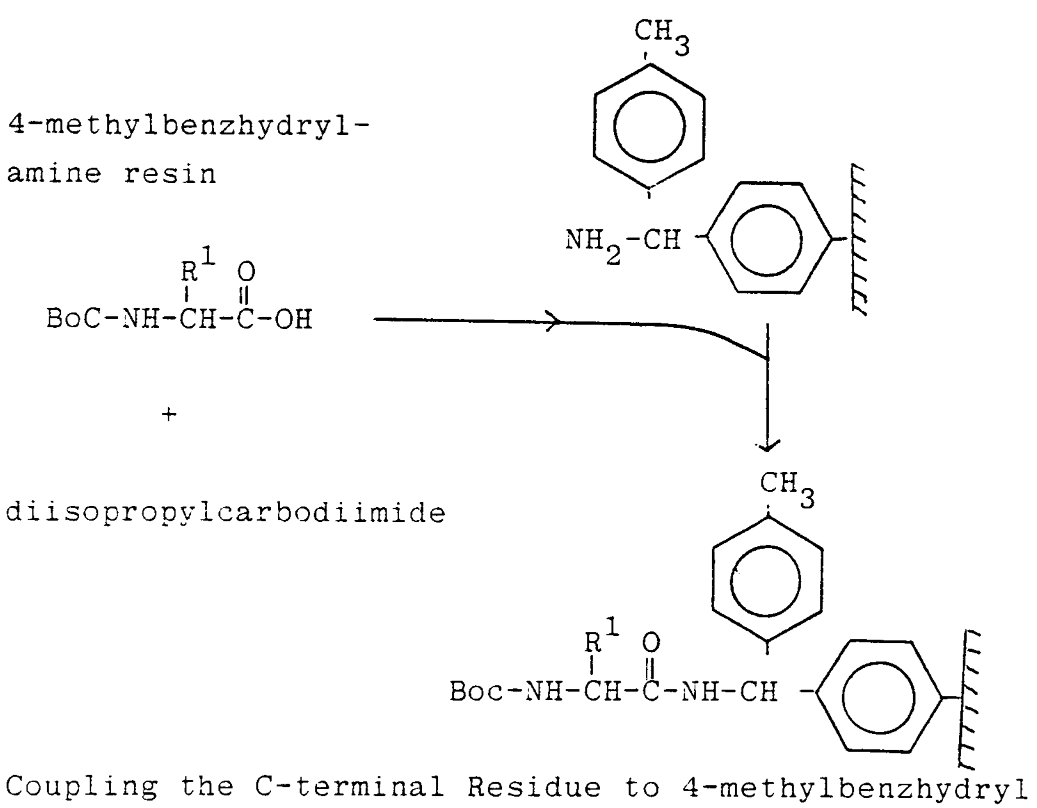


Coupling the C-terminal Residue to 4-methylbenzhydryl amine residue

The peptide synthesized according to the above described procedure is highly reactive to antibodies to HTLV-III and can be used as a highly sensitive and specific immunoadsorbent for the detection of antibodies against HTLV-III. Results obtained with the peptide according to the present invention show that

it is more sensitive and specific to antibodies against HTLV-III in body fluids than the lysate of density banded HTLV-III itself reported in U.S. Patent No. 4,520,113. (See Table II.)

Based on the high degree of sensitivity and specificity of the peptide according to the present invention in the immunoreaction to antibodies to HTLV-III, it is believed that it may also be useful as a vaccine for AIDS, ARC or pre-AIDS conditions, and as an immunogen for the development of both monoclonal and polyclonal antibodies to HTLV-III in mammals. The peptide itself or when coupled to a protein or a polymer carrier, can be introduced to normal subjects to stimulate production of antibodies to HTLV-III, and provide protection against infection by HTLV-III or LAV in healthy individuals. Since the peptide according to the present invention is not derived biochemically from the virus, there is no danger of exposing the normal subjects who are to be vaccinated to the disease.

It has been found that when the 21mer peptide conjugated to human serum albumin (HSA), as the carrier protein, was injected intraperitoneally and subcutaneously into healthy Balb/c mine, antibodies to the 21mer peptide cross reactive with HTLV-III were produced by the Balb/c mice. This is exemplified in Example 7 below.

Based on the results obtained in Example 7, the 21mer peptide can be used as an immunogen for the development of both monoclonal and polyclonal antibodies to HTLV-III in mammals.

The advantages of using the peptide according to the present invention are many.

The peptide is chemically synthesized. This means that this is no involvement with the HTLV-III virus at any time during the process of making the test reagent or the vaccine. During the preparation of the vaccine or the vaccination process, there is no risk of exposure of the production workers, individuals in the health profession and those being vaccinated to the HTLV-III virus. Similarly, there is no risk of exposure to HTLV-III in the use of the 21mer peptide for the development of monoclonal or polyclonal antibodies to HTLV-III in mammals. Further, up to the final step of the test to detect antibodies to HTLV-III, where the test reagent is exposed to the samples of sera or body fluid, there is no risk of exposure of the laboratory worker to the HTLV-III virus. Moreover, risk of exposure in this final step can be avoided by taking the precautionary step of deactivating any virus which may be present in heating at 56°C for half an hour the samples of sera or body fluids which are to be tested.

Another problem which is avoided by the process of the present invention is the possibility of false positive results caused by the presence of antigenic material from H9 cells co-purified with the HTLV-III viral preparation or E. Coli - derived proteins co-purified with expressed viral fragments. Certain normal individuals have antibodies to E. Coli or human leukocyte antigens, e.g. HLA, which are cross reactive with the antigenic materials from H9 cells. Sera samples from these normal individuals may show a positive response in the ELISA or IRMA test even though they have not been exposed to HTLV-III. A diagnosis that a person may be inflicted with AIDS based on this type of false positive response can bring severe anxiety to the person and his/her family, and cause him/her to be excluded from normal social activities. All of these problems can be avoided by using the peptide of the present invention as the test reagent.

Furthermore, with appropriate amino acid analogue substitutions, it is expected that various peptide analogues based on the prescribed amino acid sequence can be synthesized with properties giving rise to lower background readings or better adsorption capacity to solid phases useful for HTLV-III antibodies screening assays.

Various peptide analogs based on the above-described amino acid sequence have been prepared. These analogues and their reactivities with the anti HTLV-III antibodies present in sera of AIDS and ARC patients are described in Example 8 and Table VI below. The reactivity in an ELISA test for the same amount of each peptide by weight per volume was compared with the reactivity of the 21mer peptide, which was assigned a reactivity of 100.

The present results indicate that the 21mer peptide according to the present invention is unique in its size and sequence in that mere deletion of -Arg-, or -Ile- from the N- terminal or -Trp-Gly-Cys-Ser- from the C-terminal, or cleaving this peptide between -Lys- and -Asp- to form two peptides of 10 and 11 amino acids, all resulted in dramatic loss of antigenicity conferred by the 21mer peptide. In addition, -Lys- at position 12 from the C-terminal also appears to be important, in that addition of -Lys- to the above said 11mer peptide restores significantly the antigenicity.

Moreover, because the peptide in accordance with the present invention is synthetically prepared, the quality can be controlled and as a result, reproducibility of the test results can be ensured. Also, since very small amounts of peptide are required for each test procedure, and the expense of preparing the peptide is relatively low, the cost of screening body fluids for antibodies to HTLV-III and diagnosis of AIDS, ARC or pre-AIDS conditions and the preparation of a vaccine is relatively low.

The peptide prepared in accordance with the present invention can be used to detect HTLV-III infection and diagnose AIDS, ARC and pre-AIDS conditions by using it as the test reagent in an enzyme-linked

immunosorbent assay (ELISA), an enzyme immunodot assay, a hemagglutination assay or a radioimmunoradiometric assay (IRMA), preferably ELISA. The ELISA technique is exemplified in Example 1 below, the IRMA technique is exemplified in Example 3 below and the hemagglutination assay in Examples 3A and 5A below.

It is to be noted that in the following methods, 0.25%, by weight, of glutaldehyde may be added in the coating buffer to facilitate better peptide binding onto the plates or beads. Furthermore, horseradish peroxidase conjugated mouse monoclonal anti human IgG antibody may be used in place of horseradish peroxidase conjugated goat anti human IgG (Fc) as a second antibody tracer.

## EXAMPLE 1

### Detection of antibodies to HTLV-III by an enzyme-linked immunoadsorbent assay

Wells of 96-well plates were coated at 4°C overnight (or 3 hours at room temperature), with the 21mer peptide, prepared as described, at 0.5 µg per well in 100 µl 10mM $NaHCO_3$ buffer, pH 9.5. The wells were washed three times with phosphate-buffered saline (PBS) and then incubated with 250 µl of 3%, by weight, gelatin in PBS at 37°C for 1 hour to block non-specific protein binding sites, followed by three more washes with PBS containing 0.05%, by volume, Tween 20 "Tween" is a trade mark). The test sera (blood taken from a human patient or normal individual) were diluted with PBS containing 20%, by volume, normal goat serum, 1%, by weight, gelatin and 0.05%, by volume, Tween 20 at dilutions of 1:20 and 1:200, volume to volume, respectively. 200 µl of the diluted sera were added to each well and allowed to react for 1 hour at 37°C. The wells were then washed three times with 0.05%, by volume, Tween 20 in PBS in order to remove unbound antibodies. Horseradish peroxidase conjugated goat anti human IgG (Fc) was used as a second antibody tracer to bind with the AIDS antibody-antigen complex formed in positive wells. 100 µl of peroxidase labelled goat anti human IgG at a dilution of 1:3000 in 1%, by volume, normal goat serum, 0.05%, by volume, Tween 20 in PBS was added to each well and incubated at 37°C for another hour.

The wells were washed five times with 0.05%, by volume, Tween 20 in PBS to remove unbound antibody and reacted with 100 µl of the substrate mixture containing 0.04%, by weight, orthophenylenediamine (OPD) and 0.012%, by volume, hydrogen peroxide in sodium citrate buffer, pH 5.0. This substrate mixture was used to detect the peroxidase label by forming a colour product. Reactions were stopped by the addition of 50 µl of 2.5M $H_2SO_4$ and the colour yield measured using an ELISA reader which quantifies the colour reading at 492nm (i.e. OD was measured at 492nm). Assays were performed in duplicate with two sera dilutions (1:20 and 1:200, respectively). Normal serum samples were used as negative controls. Absorbance readings greater than the average readings + 4 SD (standard deviation) of 90 normal sera samples were taken as positive. The results are shown in Table I below.

TABLE I

Detection of antibodies to HTLV-III by ELISA* using 21mer peptide as solid phase immunoadsorbent

| Subject | | No. positive/ No. tested | Percent positive |
|---|---|---|---|
| 1. | Patients with AIDS | 98/100 | 98.0% |
| 2. | Patients with ARC | 90/105 | 85.7% |
| 3. | Healthy homosexuals | 17/65 | 26.2% |
| 4. | Patients with hepatoma | 0/2 | 0% |

| Subject | | No. positive/ No. tested | Percent positive |
|---|---|---|---|
| 5. | Pateints with autoimmune diseases | 0/51 | 0% |
| 6. | Patients with primary immune deficiency+ | 2/123 | 1.6% |
| 7. | Patients with lymphatic leukemias | 0/20 | 0% |
| 8. | Normal subjects | 0/90 | 0% |

* Assay was performed using sera at 1:20 (v/v) dilution with buffer.

\+ Two sera from this control patient group showed only borderline reactivity.

Note:

Sera from patients with AIDS, ARC, primary immunodeficiency, leukemia/lymphomas and normal individuals were kindly provided by Drs. S. Gupta at the University of California at Irvine, U.S.A.; S. Cunningham-Rundels at Memorial Sloan-Kettering Cancer Center Blood Bank, New York, U.S.A.; and J. Gold at the Infectious Disease Unit, Memorial Sloan-Kettering Cancer Center, New York, U.S.A. Sera from patients with autoimmune diseases including rheumatoid arthritus, systemic lupus erythematosus and allergies were kindly provided by Dr. N. Chiorazzi at the Rockerfeller University Hospital, New York, U.S.A. Sera from patients with hepatoma were provided by Dr. King-Jen Chang of the National Taiwan University Hospital.

The results in Table I show that the ELISA test procedure according to the present invention with 534 sera samples show that the method is very accurate and highly specific. No immunoreactivity was found in normal subjects or patients who were identified as not being inflicted with AIDS or ARC.

It is to be noted that in screening tests to exclude virus contaminated blood from blood banks, the criteria for defining positive reactions can be made more stringent. For example, instead of regarding average reading of normal sera samples + 4 SD as positive, it can be changed to having samples with readings equal to average reading of normal sera samples + 2 SD be regarded as positive.

EXAMPLE 2

The procedure of Example 1 was repeated using the same sera samples as in Example 1, except that the well plates were precoated with heat inactivated NP40 solubilized HTLV-III according to Gallo et al U.S. Patent No. 4,520,113. The results are presented in Table II below.

Table II

Detection of Antibodies to HTLV-III by ELISA using heat inactivated NP40 solubilized HTLV-III as solid phase immunoadsorbent

| Subject | | No. Positive/ No. Tested* | | Percent positive | |
|---|---|---|---|---|---|
| | | NS+3SD | NS-4SD | NS-3SD | NS+4SD |
| 1. | Patients with AIDS | 64/100 | 46/100 | 64.0% | 46.0% |
| 2. | Patients with ARC | 68/105 | 58/105 | 64.8% | 55.2% |
| 3. | Healthy homosexuals | 9/65 | 7/65 | 13.8% | 10.8% |
| 4. | Patients with autoimmune diseases | 3/51 | 1/51 | 5.9% | 2.0% |
| 5. | Patients with primary immune deficiency | 3/123 | 0/123 | 2.4% | 0% |
| 6. | Normal individuals* | 0/90 | 0/90 | 0% | 0% |

* Results were based on average NS (normal sera) + 3 SD and + 4SD, SD-standard deviation.

In comparison with results obtained in Example 1, this method is much less accurate and is therefore, less reliable.

EXAMPLE 3

Detection of antibodies to HTLV-III by an immunoradiometric assay (IRMA)

Wells of 96-well flexible-polyvinylchloride (PVC) plates are coated at 4°C overnight (or 3 hours at room temperature) with the 21mer peptide, prepared as described, at 0.5 µg per well in 100 µl 10mM $NaHCO_3$ buffer, pH 9.5. The wells are washed three times with phosphate-buffered saline (PBS) and then incubated with 250 µl of 3%, by weight, gelatin in PBS at 37°C for 1 hour to block non-specific protein binding sites, followed by three more washes with PBS containing 0.05%, by volume, Tween 20. The test sera (blood taken from a human patient or normal individual) are diluted with PBS containing 20%, by volume, normal goat serum, 1%, by weight, gelatin and 0.05%, by volume, Tween 20 at dilutions of 1:20 and 1:200 (volume to volume), respectively. 200 µl of the diluted sera are added to each well and allowed to react for 1 hour at 37°C. The wells are then washed three times with 0.05%, by volume, Tween 20 in PBS in order to remove unbound antibodies. $I^{125}$ labelled affinity purified goat anti human IgG (Fc) is used as a second antibody tracer that binds with the antibody-antigen complex formed in positive wells. 100 µl of $I^{125}$ labelled goat anti human IgG of 50,000-200,000 cpm in 1%, by volume, normal goat serum, 0.05%, by volume, Tween 20 in PBS was added to each well and incubated at 37°C for another hour.

The wells are washed five times with 0.05%, by volume, Tween 20 in PBS to remove unbound second antibody and dried. The wells are cut and counted by a gamma-scintillation counter. Assays are performed in duplicate with two sera dilutions, 1:20 and 1:200, volume to volume, respectively. Normal sera sample as negative controls were also tested simultaneously. Cpm readings greater than the average readings of normal sera samples +4 SD (standard deviation) are taken as positive.

EXAMPLE 3A

Detection of antibodies to HTLV-III by a hemagglutination assay using 21-mer peptide coated gelatin particles, sheep red blood cells or latex beads as solid phase immunoadsorbent

1 ml thoroughly washed sheep red blood cells, gelatin particles, or polystyrene latex beads are coated with the 21mer peptide at a concentration in the range of 5µg/ml to 1m mg/ml. The 21mer peptide coated cells, particles or beads are then incubated with serially diluted serum samples in the wells of a 96-well U-shaped microplate. After being left at room temperature for about an hour, the agglutination patterns on the bottom are read, and the largest dilution showing a positive reaction is recorded.

This is a one-step assay which could be used for both qualitative and quantitative analysis of the presence of anti-HTLV-II antibodies in specimens including sera or biofluids.

EXAMPLE 4

A diagnostic AIDS, ARC and pre-AIDS specific test kit for HTLV-III antibodies detection can be constructed. The test kit comprises a compartmented enclosure containing multiple 96-well plates coated prior to use with 0.5 µg of the peptide according to the present invention in 100 µl pH 9.5 10mM $NaHCO_3$ buffer per well. The kit further comprises materials for enzyme detection in spearate sealed containers comprising: (1) normal human serum (as negative control); (2) heat-inactivated, NP40-solubilized AIDS serum (as positive control); (3) normal goat serum; (4) peroxidase labelled-goat anti human IgG; and (5) a colour change indicator comprising orthophenylenediamine (OPD) and hydrogen peroxide in phosphate citrate buffer. The procedure described in Example 1 is to be followed.

In this test kit, 96-well plates, precoated with the peptide in accordance with the present invention, can be replaced by polystyrene beads, or multiple mini-columns filled with controlled pore size glass beads, or nitrocellulose paper strip precoated with the peptide according to the present invention for use as the solid phase immunoadsorbent.

EXAMPLE 5

A second test kit for detecting antibodies using the immunoradiometric assay (IRMA) comprised a compartmented enclosure containing multiple 96-well bendable polyvinylchloride (PVC) plates precoated with peptide according to the present invention at a concentration of 0.5 µg of peptide in 100 µl of pH 9.5 10mM $NaHCO_3$ buffer per well and materials for radioimmunoassay including: (1) normal human serum (as negative control); (2) heat-inactivated, NP40-solubilized AIDS serum (as positive control); (3) normal goat serum; and, (4) $I^{125}$ labelled goated anti human IgG. The procedure described in Example 3 is to be followed.

In this test kit, 96-well PVC plates precoated with the peptide in accordance with the present invention can be replaced by polystyrene beads precoated with the peptide according to the present invention for use as the solid phase immunoadsorbent.

EXAMPLE 5A

A third test kit for detecting HTLV-III antibodies using the hemagglutination assay comprised a compartmented enclosure containing multiple 96-well U-shaped microplates amd materials for hemagglutination assay including: (1) a bottle of 21-mer peptide coated sheep red blood cells, gelatin particles or latex polystyrene beads; (2) normal human serum (as a negative control); and, (3) heat-inactivated, NP40-solubilized AIDS positive serum (as positive control). The procedure described in Example 3A is to be followed.

EXAMPLE 6

An experiment was conducted to compare AIDS screening results using the method of Example 1 and heat-inactivated, NP40-solubilized HTLV-III according to U.S. Patent No. 4,520,113. Sera from normal subjects, patients with AIDS, ARC, were diluted (1:20 and 1:200). Duplicates of each diluted sera sample were tested against the peptide according to the present invention and cultured HTLV-III according to U.S. Patent 4,520,113. Normal human serum and heat-inactivated HTLV-III reactive AIDS serum samples were used as control. The results are shown in Table III below.

TABLE III

Comparison of OD at 492 nm obtained by using peptide according to the present invention and heat-inactivated NP40-solubilized HTLV III as solid phase immunoadsorbent

| Dilutions of human sera: | | 1/20 (v:v) | | 1/200 (v:v) | |
|---|---|---|---|---|---|
| Sample No. | Subjects | Peptide | HTLV-III | Peptide | HTLV-III |
| | AIDS control | over* | 1.64±.35 | over | 0.67±.08 |
| | Normal pooled serum | 0.43±.02 | 0.35±.02 | 0.10±0 | 0.08±0 |
| 335 | AIDS | 1.51±.10 | 0.46±.02 | 0.23±.02 | 0.22±0 |
| 336 | AIDS | 0.96±.08 | 0.45±.06 | 0.13±.01 | 0.15±.01 |
| 337 | NS** | 0.34±.02 | 0.21±.01 | 0.05±.0 | 0.05±.01 |
| 338 | NS | 0.30±0 | 0.36±.04 | 0.08±.01 | 0.08±.01 |
| 339 | NS | 0.29±.03 | 0.29±.01 | 0.06±.01 | 0.07±.01 |
| 340 | AIDS | 1.65±.06 | 0.37±.03 | 0.22±.04 | 0.18±.01 |
| 341 | AIDS | over | 0.83±.05 | 0.36±.01 | 0.30±.01 |
| 344 | NS | 0.14±0 | 0.14±.02 | 0.03±0 | 0.07±.01 |
| 345 | AIDS | over | 0.62±.08 | 0.30±0 | 0.25±0 |
| 346 | AIDS | over | 1.72±.11 | 0.79±.01 | 0.48±.02 |
| 347 | AIDS | over | 0.54±.01 | 0.77±.04 | 0.22±0 |
| 348 | AIDS | 1.70±.01 | 1.07±.01 | 0.23±.01 | 0.30±.01 |
| 349 | NS | 0.24±.01 | 0.26±0 | 0.24±0 | 0.10±.01 |
| 350 | AIDS | over | 1.35±.05 | over | 0.44±.04 |
| 351 | NS | 0.23±.01 | 0.25±0 | 0.05±.01 | 0.10±.04 |
| 352 | NS | 0.24±.01 | 0.23±0 | 0.04±.01 | 0.04±.01 |
| 356 | AIDS | 1.58±.16 | 0.73±.08 | 0.18±0 | 0.26±.01 |
| 357 | AIDS | over | 0.42±.02 | 1.63±.02 | 0.20±.01 |
| 358 | AIDS | over | 1.50±.13 | over | 0.82±.08 |
| 359 | NS | 0.28±.01 | 0.22±.01 | 0.09±.01 | 0.08±.01 |
| 360 | NS | 0.10±.01 | 0.10±.01 | 0.02±.01 | 0.04±.03 |
| 361 | AIDS | over | 0.60 | 1.71 | 0.25 |

* over = $OD_{492nm}$ reading higher than 2.0

** NS = normal serum

The results in Table III show that the method is highly sensitive. The optical density achieved using the peptide in accordance with the present invention against duplicate AIDS sera samples at the same dilution is much higher than the optical density achieved using deactivated HTLV-III against identical sera samples at identical dilutions. Furthermore, where the results with deactivated HTLV-III may be questionable, e.g. Samples 335, 336, 340, 345, 347, 357 and 361, results with the peptide in accordance with the present invention is definitive, demonstrating the high degree of sensitivity and specificity of the method.

EXAMPLE 7

Four healthy Balb/c mice were bled and their sera tested according to the procedure of Examples 1 and 2. The results indicated that the Balb/c mice were free of antibodies to both HTLV-III and the 21mer peptide as shown in Table IV below. The four mice were then separated into two groups A and B and immunized according to the following procedures.

GROUP A

Two of the Balb/c mice were injected with sucrose-banded HTLV-III virus mixed with an equal volume of complete Freund's adjuvant at 20 µg/0.2 ml/injection into multiple sites both intraperitoneally and subcutaneously. The immunization was repeated according to the following schedule:

| | days |
|---|---|
| initial immunization | 0 |
| second immunization | 14 |
| third immunization | 21 |
| fourth immunization | 28 |
| fifth immunization | 35 |
| sixth immunization | 42 |

GROUP B

Two of the Balb/c mice were similarly injected with the 21mer peptide conjugated to human serum albumin (HSA), as the carrier protein, at 20 µg/20 µg HSA/0.2ml/injection. The immunization schedule was also the same as that of Group A.

Bleedings were taken from both Group A and B after the sixth immunization and the sera analyzed by the procedures of Examples 1 and 2, except using horseradish peroxidase conjugated goat anti mouse IgG as the tracer antibody. The results are shown in Table V below.

The results indicate that the 21mer peptide represents a highly immunogenic epitope present on HTLV-III since sera obtained from mice previously immunized with HTLV-III contain high titer antibodies to HTLV-III as well as to the 21mer peptide. Furthermore, the 21mer peptide is a potent immunogen in eliciting the production of antibodies to HTLV-III in healthy mammals as demonstrated by the presence of high titer antibodies to HTLV-III in the sera obtained from mice previously immunized with the 21mer peptide coupled to human serum albumin as a carrier protein.

It is quite remarkable that synthetic peptides representing minute fractions of a large virus can be manipulated to mimic the antigenic/immunogenic process to an extent that neutralizing or protective antibodies can be prepared from these synthetic peptides. The potential advantages in cost and safety of using the synthetic peptides as vaccines make this approach a realistic expectation. The 21mer peptide, its analogues and segments, as described herein, have been found to represent highly immunogenic epitopes of HTLV-III in that the 21mer peptide, along with its conjugated forms, can elicit high titers of antibodies to HTLV-III has hereby been demonstrated and are, therefore, candidates for use as synthetic vaccines in mammals or humans to elicit specific antibodies to HTLV-III for protective purposes. It is also expected that polymeric forms of the 21mer peptide, its analogues and segments, will also be useful as candidates for synthetic vaccines for the same purposes.

TABLE IV

Unimmunized normal Balb/C mice

$OD_{492}$

| Coating antigen | Undiluted | Serum titer 1/10 | 1/100 | 1/1000 | 1/10,000 | 1/100,000 | B.G.* |
|---|---|---|---|---|---|---|---|
| HTLV-III (10µg/ml) | 0.17 | 0.06 | 0.04 | 0.05 | 0.05 | 0.01 | 0.06 |
| 21mer (5 µg/ml) | 0.15 | 0.07 | 0.04 | 0.03 | 0.05 | 0.02 | 0.04 |

TABLE V

GROUP A

Balb/C mice immunized with HTLV-III virus

$OD_{492}$

| Coating antigen | Serum titer 1/10 | 1/100 | 1/1000 | 1/10,000 | 1/100,000 |
|---|---|---|---|---|---|
| HTLV-III (10µg/ml) | 2.0 | 2.0 | 0.76 | 0.25 | 0.16 |
| 21mer peptide (5µg/ml) | 2.0 | 2.0 | 0.52 | 0.25 | 0.13 |

GROUP B

Balb/C mice immunized with 21mer peptide

$OD_{492}$

| Coating antigen | Serum titer 1/10 | 1/100 | 1/500 | 1/1000 | 1/2500 | B.G.* |
|---|---|---|---|---|---|---|
| HTLV-III (10µg/ml) | 2.24 | 1.64 | 0.97 | 0.75 | 0.56 | 0.05 |
| 21mer peptide (5µg/ml) | 2.12 | 0.48 | 0.12 | 0.11 | 0.10 | 0.06 |

* B.G. = background

EXAMPLE 8

Eight analogues of the 21mer peptide and an overlapping 19mer peptide were prepared in their amide form according to the classical Merrifield method described above. The amino acid sequence of each of the analogues are shown in Table VI below.

A serum sample from a patient diagnosed as having AIDS and previously determined to contain antibodies to HTLV-III was obtained. ELISA tests according to Example 1 were conducted using each of the analogues as the coating antigen against the above AIDS serum sample. The reactivity of each of the analogues was compared with that of the 21mer peptide which was assigned a value of 100. The results are shown in Table VI below.

TABLE VI

| Peptide | Amino acid sequence | Relative % Reactivity |
|---|---|---|
| 1. | Val-Trp-Gly-Ile-Lys-Gln-Leu-Gln-Ala-Arg-Ile-Leu-Ala-Val-Glu-Arg-Tyr-Leu-Lys | 5 |
| 2. | Gln-Leu-Gln-Ala-Arg-Ile-Leu-Ala-Val-Glu-Arg-Tyr-Leu-Lys | 13.5 |
| 3. | Arg-Ile-Leu-Ala-Val-Glu-Arg-Tyr-Leu-Lys | 5 |
| 4. | Arg-Ile-Leu-Ala-Val-Glu-Arg-Tyr-Leu-Lys-Asp-Gln-Gln-Leu-Leu-Gly-Ile | 28.3 |
| 5. | Ile-Leu-Ala-Val-Glu-Arg-Tyr-Leu-Lys-Asp-Gln-Gln-Leu-Leu-Gly-Ile | 30.3 |
| 6. | Arg-Ile-Leu-Ala-Val-Glu-Arg-Tyr-Leu-Lys-Asp-Gln-Gln-Leu-Leu-Gly-Ile-Trp-Gly-Cys-Ser | 100 |
| 7. | Arg-Leu-Ala-Val-Glu-Arg-Tyr-Leu-Lys-Asp-Gln-Gln-Leu-Leu-Gly-Ile-Trp-Gly-Cys-Ser | 66.8 |
| 8. | Val-Glu-Arg-Tyr-Leu-Lys-Asp-Gln-Gln-Leu-Leu-Gly-Ile-Trp-Gly-Cys-Ser | 53.9 |
| 9. | Lys-Asp-Gln-Gln-Leu-Leu-Gly-Ile-Trp-Gly-Cys-Ser | 38.3 |
| 10. | Asp-Gln-Gln-Leu-Leu-Gly-Ile-Trp-Gly-Cys-Ser | 8 |
| 11. | Ile-Leu-Ala-Val-Glu-Arg-Tyr-Leu-Lys-Asp-Gln-Gln-Leu-Leu-Gly-Ile-Trp-Gly-Cys-Ser | 20.0 |

TABLE VII

Calculation of % reactivity

| $OD_{492}$* Coating peptide concentration | 5 μg/ml | | 20 μg/ml | | 80 μg/ml | | % Reactivity |
|---|---|---|---|---|---|---|---|
| Serum dilution | 1/200 AIDS | 1/400 AIDS | 1/200 | 1/400 | 1/200 | 1/400 | $\sqrt{\frac{\sum_{i=1}^{6} OD_{492\,i}^2}{6}}$ |
| Analogue peptide | i=1 | i=2 | i=3 | i=4 | i=5 | i=6 | i=6 |
| 6 | 1 | 1 | 1 | 1 | 1 | 1 | 100% |
| 7 | 0.60 | 0.64 | 0.67 | 0.70 | 0.73 | 0.66 | 66.8% |
| 8 | 0.45 | 0.49 | 0.59 | 0.53 | 0.63 | 0.53 | 53.9% |
| 9 | 0.21 | 0.20 | 0.38 | 0.39 | 0.53 | 0.50 | 38.9% |
| 10 | 0.01 | 0 | 0.07 | 0.04 | 0.14 | 0.11 | 8.0% |
| 11 | 0.12 | 0.09 | 0.23 | 0.21 | 0.28 | 0.23 | 20.0% |
| 4 | 0.34 | 0.25 | 0.30 | 0.24 | 0.31 | 0.24 | 28.3% |
| 5 | 0.30 | 0.22 | 0.30 | 0.26 | 0.40 | 0.31 | 30.3% |
| 3 | 0.01 | 0.02 | 0.01 | 0.01 | 0.07 | 0.06 | 3.9% |
| 2 | 0.01 | 0.02 | 0.06 | 0.05 | 0.25 | 0.20 | 13.5% |

$$*OD_{492} = \frac{OD_{492} \text{ reading on an analogue peptide coated well}}{OD_{492} \text{ reading on 21mer peptide coated well}}$$

## Claims

**Claims for the following Contracting States : BE, CH, DE, FR, GB, IT, LI, LU, NL, SE**

**1.** A peptide composition having specific immunoreactivity to antibodies to the AIDS virus characterised in that it is selected from peptides containing from twelve to twenty-one amino acids, the sequence of which corresponds to a part or the entirety of the following:

Arg-Ile-Leu-Ala-Val-Glu-Arg-Tyr-Leu-Lys-Asp-Gln-Gln-Leu-Leu-Gly-Ile-Trp-Gly-Cys-Ser;

analogues thereof wherein the amino acids in the sequence are substituted, deleted or added as long as the immunoreactivity to antibodies to the AIDS virus is preserved; and conjugates of the peptides or analogues thereof.

2. A peptide as claimed in claim 1 wherein one or more amino acids is/are deleted from either terminus of the amino acid sequence.

3. A peptide as claimed in claim 2 wherein it comprises at least fifteen amino acids.

4. A peptide chemically synthesized in vitro characterised in that it comprises an amino acid sequence substantially corresponding to an antigenic amino acid sequence present in the envelope protein of the AIDS virus, the amino acid sequence taken from the left to right and in the direction from the N-terminus to the C-terminus comprising one of the following amino acid sequences:
(a) -Trp-Gly-Cys-Ser-;
(b) -Asp-Gln-Gln-Leu-Leu-Gly-Ile-;
and
(c) -Ile-Leu-Ala-Val-Glu-Arg-Tyr-Leu-Lys-.

5. A method for the detection of antibodies to the AIDS virus and the diagnosis of AIDS, ARC and pre-AIDS conditions characterised in that it comprises:
(a) preparing a peptide as claimed in claim 1 or claim 4;
and
(b) using an effective amount of the peptide as coating antigen to detect antibodies to the AIDS virus in an immunoassay procedure.

6. A method as claimed in claim 5 wherein the immunoassay procedure is an ELISA, IRMA, hemagglutination or multidot strip assay procedure.

7. A method as claimed in claim 6 wherein, for each test, from about 0.1 to about 20 $\mu$g of the peptide in a buffer at a pH of from about 7 to about 10 is used as the antigen.

8. A method as claimed in claim 6 wherein, for each test, about 1 $\mu$g of the peptide in a buffer at a pH of from about 7 to about 10, is used as the antigen.

9. A method as claimed in claim 7 wherein the buffer is a 10mM bicarbonate buffer at a pH of from about 9 to about 10.

10. A method as claimed in claim 9 wherein the buffer is a 10mM bicarbonate buffer at a pH of about 9.5.

11. A method as claimed in claim 7 wherein the buffer is a 50mM TRIS buffer at a pH of from about 7 to about 8.

12. A method as claimed in claim 11 wherein the buffer is a 50mM TRIS buffer at a pH of about 7.5.

13. A test kit for the detection of antibodies to the AIDS virus and the diagnosis of AIDS, ARC and pre-AIDS conditions characterised in that the immunoadsorbent comprises a peptide composition as claimed in claim 1 or claim 4.

14. A vaccine for eliciting the production of antibodies to the AIDS virus in mammals characterised in that it comprises a peptide as claimed in claim 1 or claim 4.

15. A vaccine as claimed in claim 14 wherein the peptide is a polymer thereof.

16. A vaccine as claimed in claim 14 wherein the peptide is coupled to a protein carrier.

17. A vaccine as claimed in claim 16 wherein the peptide is conjugated to human serum albumin.

18. A vaccine as claimed in claim 14 wherein the peptide is coupled to a polymer.

19. An immunogen for the development of monoclonal or polyclonal antibodies to the AIDS virus in mammals characterised in that there is used a peptide as claimed in claim 1 or claim 4.

**20.** An immunogen as claimed in claim 19 wherein the peptide is a polymer thereof.

**21.** An immunogen as claimed in claim 19 wherein the peptide is coupled to a protein carrier.

**22.** An immunogen as claimed in claim 21 wherein the peptide is conjugated to human serum albumin.

**23.** An immunogen according to claim 19 wherein the peptide is coupled to a polymer.

**24.** A method for the production of monoclonal or polyclonal antibodies to the AIDS virus in mammals characterised in that it comprises using an immunogen as claimed in any of claims 19 to 23.

**25.** Monoclonal or polyclonal antibodies against the AIDS virus characterised in that they are prepared by a method as claimed in claim 24.

**26.** A peptide as claimed in claim 1 wherein it is:

Arg-Ile-Leu-Ala-Val-Glu-Arg-Tyr-Leu-Lys-Asp-Gln-Gln-
Leu-Leu-Gly-Ile-Trp-Gly-Cys-Ser.

**27.** A test kit for the detection of antibodies to the AIDS virus and the diagnosis of AIDS, ARC and pre-AIDS conditions characterised in that it comprises:
(a) a solid support;
(b) coated onto the solid support, an immunoadsorbent comprising a peptide composition as claimed in claim 1 or claim 4 or claim 26;
(c) a sample of normal serum as negative control;
(d) a sample of serum containing antibodies to the AIDS virus as a positive control; and
(e) a buffer for diluting the serum samples.

**Claims for the following Contracting State : AT**

**1.** A process for the production of a peptide composition having specific immunoreactivity to antibodies to the AIDS virus characterised in that there is chemically synthesized a peptide selected from peptides containing twelve to twenty-one amino acids, the sequence of which corresponds to a part or the entirety of the following:

Arg-Ile-Leu-Ala-Val-Glu-Arg-Tyr-Leu-Lys-Asp-Gln-Gln-Leu-Leu-
Gly-Lle-Trp-Gly-Cys-Ser;

analogues thereof wherein the amino acids in the sequence are substituted, deleted or added as long as the immunoreactivity to antibodies to the AIDS virus is preserved; and conjugates of the peptides or analogues thereof.

**2.** A process as claimed in claim 1 wherein one or more amino acids is/are deleted from either terminus of the amino acid sequence.

**3.** A process as claimed in claim 2 wherein the peptide produced comprises at least fifteen amino acids.

**4.** A process for the production of a peptide in vitro characterised in that it comprises chemically synthesizing an amino acid sequence substantially corresponding to an antigenic amino acid sequence present in the envelope protein of the AIDS virus, the amino acid sequence produced taken from left to right and in the direction from the N-terminus to the C-terminus comprises one of the following amino acid sequences:
(a) -Trp-Gly-Cys-Ser;

(b) -Asp-Gln-Gln-Leu-Leu-Gly-Ile-;
and
(c) -Ile-Leu-Ala-Val-Glu-Arg-Tyr-Leu-Lys-.

**5.** A method for the detection of antibodies to the AIDS virus and the diagnosis of AIDS, ARC or pre-AIDS conditions charaterised in that it comprises:
(a) preparing a peptide by a process as claimed in claim 1 or claim 4; and
(b) using an effective amount of the peptide as a coating antigen to detect antibodies to the AIDS virus in an immunoassay procedure.

**6.** A method as claimed in claim 5 wherein the immunoassay procedure is an ELISA, IRMA, hemagglutination or multidot strip assay procedure.

**7.** A method as claimed in claim 6 wherein, for each test, from about 0.1 to about 20 µg of the peptide in a buffer at a pH of from about 7 to about 10 is used as the antigen.

**8.** A method as claimed in claim 6 wherein, for each test, about 1 µg of the peptide in a buffer at a pH of from about 7 to about 10, is used as the antigen.

**9.** A test kit for the detection of antibodies to the AIDS virus and the diagnosis of AIDS, ARC and pre-AIDS conditions characterised in that the immunoadsorbent comprises a peptide composition produced by a process as claimed in claim 1 or claim 4.

**10.** A process for the production of a vaccine for eliciting the production of antibodies to the AIDS virus mammals characterised in that it comprises the use of a peptide produced by a process as claimed in claim 1 or claim 4.

**11.** A process as claimed in claim 11 wherein the peptide used is a polymer thereof.

**12.** A process as claimed in claim 10 wherein the peptide used is coupled to a protein carrier.

**13.** A process as claimed in claim 12 wherein the peptide used is conjugated to human serum albumin.

**14.** A process as claimed in claim 10 wherein the peptide used is coupled to a polymer.

**15.** A process for the production of an immunogen for the development of monoclonal or polyclonal antibodies to the AIDS virus in mammals characterised in that there is used a peptide produced by a process as claimed in claim 1 or claim 4.

**16.** A process as claimed in claim 15 wherein the peptide used is a polymer thereof.

**17.** A process as claimed in claim 15 wherein the peptide used is coupled to a protein carrier.

**18.** A process as claimed in claim 17 wherein the peptide used is conjugated to human serum albumin.

**19.** A process as claimed in claim 15 wherein the peptide used is coupled to a polymer.

**20.** A method for the production of monoclonal or polyclonal antibodies to the AIDS virus in mammals characterised in that it comprises using an immunogen produced by a process as claimed in any of claims 15 to 19.

**21.** A process for the production of a peptide as claimed in claim 1 wherein it is:

Arg-Ile-Leu-Ala-Val-Glu-Arg-Tyr-Leu-Lys-Asp-Gln-Gln-
Leu-Leu-Gly-Ile-Trp-Gly-Cys-Ser.

**22.** A test kit for the detection of antibodies to the AIDS virus and the diagnosis of AIDS, ARC and pre-AIDS conditions characterised in that it comprises:
(a) a solid support;
(b) coated onto the solid support, an immunoadsorbent comprising a peptide composition produced by a process as claimed in claim 1 or claim 4 or claim 21;
(c) a sample of normal serum as negative control;
(d) a sample of serum containing antibodies to the AIDS virus as a positive control; and
(e) a buffer for diluting the serum samples.

## Patentansprüche

**Patentansprüche für folgende Vertragsstaaten : BE, CH, DE, FR, GB, IT, LI, LU, NL, SE**

**1.** Peptidzusammensetzung mit spezifischer Immunoreaktivität gegenüber Antikörpern des AIDS-Virus, **dadurch gekennzeichnet,** daß
sie aus zwölf bis einundzwanzig Aminosäuren enthaltenden Peptiden ausgewählt ist, deren Sequenz einem Teil oder der Gesamtheit der folgenden:

Arg-Ile-Leu-Ala-Val-Glu-Arg-Tyr-Leu-Lys-Asp-Gln-Gln-
Leu-Leu-Gly-Ile-Trp-Gly-Cys-Ser

entspricht, Analogons davon, worin die Aminosäuren in der Sequenz substituiert, entfernt oder hinzugefügt werden, solange die Immunoreaktivität gegenüber den Antikörpern des AIDS-Virus erhalten ist, und Konjugaten der Peptide oder Analogons davon.

**2.** Peptid nach Anspruch 1, worin eine oder mehrere Aminosäure(n) von jedem Ende der Aminosäuresequenz entfernt sind.

**3.** Peptid nach Anspruch 2, worin es zumindest fünfzehn Aminosäuren enthält.

**4.** In vitro chemisch synthetisiertes Peptid,
**dadurch gekennzeichnet,** daß
es eine Aminosäuresequenz enthält, welche im wesentlichen einer im Oberflächenprotein des AIDS-Virus vorliegenden antigenen Aminosäuresequenz entspricht, wobei die Aminosäuresequenz von links nach rechts und in Richtung vom N-Terminus zum C-Terminus eine der folgenden Aminosäuresequenzen:
(a) -Trp-Gly-Cys-Ser-,
(b) -Asp-Gln-Gln-Leu-Leu-Gly-Ile- und
(c) -Ile-Leu-Ala-Val-Glu-Arg-Tyr-Leu-Lys-
enthält.

**5.** Verfahren zum Bestimmen von Antikörpern des AIDS-Virus und zur Diagnose von AIDS-, ARC- und pre-AIDS-Zuständen,
**dadurch gekennzeichnet,** daß
(a) ein Peptid gemäß Anspruch 1 oder Anspruch 4 hergestellt und
(b) eine wirksame Menge des Peptids als Coating-Antigen eingesetzt wird, um Antikörper gegen das AIDS-Virus in einem Immunoassay-Verfahren zu bestimmen.

**6.** Verfahren nach Anspruch 5, worin das Immunoassay-Verfahren ein ELISA-, IRMA-, Haemagglutinations- oder Multidot-Strip-Assay-Verfahren ist.

**7.** Verfahren nach Anspruch 6, worin für jeden Test von etwa 0,1 bis etwa 20 µg des Peptids in einem Puffer bei einem pH-wert von etwa 7 bis etwa 10 als Antigen verwendet wird.

**8.** Verfahren nach Anspruch 6, worin für jeden Test etwa 1 µg des Peptids in einem Puffer bei einem pH-Wert von etwa 7 bis etwa 10 als Antigen verwendet wird.

9. Verfahren nach Anspruch 7, worin als Puffer ein 10 mM Bicarbonatpuffer mit einem pH-Wert von etwa 9 bis etwa 10 verwendet wird.

10. Verfahren nach Anspruch 9, worin der Puffer ein 10 mM Bicarbonatpuffer mit einem pH-Wert von etwa 9,5 ist.

11. Verfahren nach Anspruch 7, worin der Puffer ein 50 mM TRIS-Puffer mit einem pH-Wert von etwa 7 bis etwa 8 ist.

12. Verfahren nach Anspruch 11, worin der Puffer ein 50 mM TRIS-Puffer mit einem pH-Wert von etwa 7,5 ist.

13. Testkit zum Bestimmen von Antikörpern des AIDS-Virus und zur Diagnose von AIDS-, ARC- und pre-AIDS-Zuständen,
**dadurch gekennzeichnet,** daß
das Immunoadsorbens eine Peptidzusammensetzung nach Anspruch 1 oder Anspruch 4 enthält.

14. Vakzin zum Auffinden der Antikörperproduktion des AIDS-Virus in Säugetieren,
**dadurch gekennzeichnet,** daß
es ein Peptid nach Anspruch 1 oder Anspruch 4 enthält.

15. Vakzin nach Anspruch 14, worin das Peptid ein Polymer davon ist.

16. Vakzin nach Anspruch 14, worin das Peptid an einen Proteincarrier gekoppelt ist.

17. Vakzin nach Anspruch 16, worin das Peptid mit Humanserumalbumin konjugiert ist.

18. Vakzin nach Anspruch 14, worin das Peptid an ein Polymer gekoppelt ist.

19. Immunogen zur Bildung monoklonaler oder polyklonaler Antikörper gegen das AIDS-Virus in Säugetieren,
**dadurch gekennzeichnet,** daß
ein Peptid nach Anspruch 1 oder Anspruch 4 verwendet wird.

20. Immunogen nach Anspruch 19, worin das Peptid ein Polymer davon ist.

21. Immunogen nach Anspruch 19, worin das Peptid an einen Proteincarrier gekoppelt ist.

22. Immunogen nach Anspruch 21, worin das Peptid mit Humanserumalbumin konjugiert ist.

23. Immunogen nach Anspruch 19, worin das Peptid an ein Polymer gekoppelt ist.

24. Verfahren zum Herstellen monoklonaler oder polyklonaler Antikörper gegen das AIDS-Virus in Säugetieren,
**dadurch gekennzeichnet,** daß
ein Immunogen nach einem der Ansprüche 19 bis 23 verwendet wird.

25. Monoklonale oder polyklonale Antikörper gegen das AIDS-Virus,
**dadurch gekennzeichnet,** daß
sie durch ein Verfahren nach Anspruch 24 hergestellt werden.

26. Peptid nach Anspruch 1, worin es:

Arg-Ile-Leu-Ala-Val-Glu-Arg-Tyr-Leu-Lys-Asp-Gln-Gln-
Leu-Leu-Gly-Ile-Trp-Gly-Cys-Ser

ist.

**27.** Testkit zum Bestimmen von Antikörpern des AIDS-Virus und zur Diagnose von AIDS-, ARC- und pre-AIDS-Zuständen,
**dadurch gekennzeichnet,** daß
es
(a) einen festen Träger,
(b) ein auf einen festen Träger aufgebrachtes, eine Peptidzusammensetzung nach Anspruch 1 oder Anspruch 4 oder Anspruch 26 enthaltendes Immunoadsorbens ,
(c) eine Probe eines Normalserums als Negativkontrolle,
(d) eine Probe eines Antikörper dsn AIDS-Virus enthaltenden Serums als Positivkontrolle und
(e) einen Puffer zum Verdünnen der Serumproben
enthält.

**Patentansprüche für folgenden Vertragsstaat : AT**

**1.** Verfahren zum Herstellen einer Peptidzusammensetzung mit spezifischer Immunoreaktivität gegenüber Antikörpern des AIDS-Virus,
**dadurch gekennzeichnet,** daß
ein Peptid, das aus zwölf bis einundzwanzig Aminosäuren enthaltenden Peptiden ausgewählt ist, deren Sequenz einem Teil oder der Gesamtheit der folgenden:

Arg-Ile-Leu-Ala-Val-Glu-Arg-Tyr-Leu-Lys-Asp-Gln-Gln-
Leu-Leu-Gly-Ile-Trp-Gly-Cys-Ser

entspricht, Analogons davon, worin die Aminosäuren in der Sequenz substituiert, entfernt oder hinzugefügt werden, solange die Immunoreaktivität gegenüber den Antikörpern des AIDS-Virus erhalten ist, und Konjugaten der Peptide oder Analogons davon, chemisch synthetisiert wird.

**2.** Verfahren nach Anspruch 1, worin eine oder mehrere Aminosäure(n) von jedem Ende der Aminosäuresequenz entfernt werden.

**3.** Verfahren nach Anspruch 2, worin das hergestellte Peptid zumindest fünfzehn Aminosäuren enthält.

**4.** Verfahren zum Herstellen eines in vitro chemisch synthetisierten Peptids,
**dadurch gekennzeichnet,** daß
es die chemische Synthese einer Aminosäuresequenz umfaßt, welche im wesentlichen einer im Oberflächenprotein des AIDS-Virus vorliegenden antigenen Aminosäuresequenz entspricht, wobei die Aminosäuresequenz von links nach rechts und in Richtung vom N-Terminus zum C-Terminus eine der folgenden Aminosäuresequenzen:
(a) -Trp-Gly-Cys-Ser-,
(b) -Asp-Gln-Gln-Leu-Leu-Gly-Ile- und
(c) -Ile-Leu-Ala-Val-Glu-Arg-Tyr-Leu-Lys-
enthält.

**5.** Verfahren zum Bestimmen von Antikörpern des AIDS-Virus und zur Diagnose von AIDS-, ARC- und pre-AIDS-Zuständen,
**dadurch gekennzeichnet,** daß
(a) ein Peptid gemäß einem Verfahren nach Anspruch 1 oder Anspruch 4 hergestellt und
(b) eine wirksame Menge des Peptids als Coating-Antigen eingesetzt wird, um Antikörper gegen das AIDS-Virus in einem Immunoassay-Verfahren zu bestimmen.

**6.** Verfahren nach Anspruch 5, worin das Immunoassay-Verfahren ein ELISA-, IRMA-, Haemagglutinations- oder Multidot-Strip-Assay-Verfahren ist.

**7.** Verfahren nach Anspruch 6, worin für jeden Test von etwa 0,1 bis etwa 20 µg des Peptids in einem Puffer bei einem pH-Wert von etwa 7 bis etwa 10 als Antigen verwendet wird.

**8.** Verfahren nach Anspruch 6, worin für jeden Test etwa 1 µg des Peptids in einem Puffer bei einem pH-Wert von etwa 7 bis etwa 10 als Antigen verwendet wird.

**9.** Testkit zum Bestimmen von Antikörpern des AIDS-Virus und zur Diagnose von AIDS-, ARC- und pre-AIDS-Zuständen,
**dadurch gekennzeichnet,** daß
das Immunoadsorbens eine nach einem Verfahren gemäß Anspruch 1 oder Anspruch 4 hergestellte Peptidzusammensetzung enthält.

**10.** Verfahren zum Herstellen eines Vakzins zum Auffinden der Antikörperproduktion des AIDS-Virus in Säugetieren,
**dadurch gekennzeichnet,** daß
es die Verwendung eines durch ein Verfahren nach Anspruch 1 oder Anspruch 4 hergestellten Peptids umfaßt.

**11.** Verfahren nach Anspruch 11, worin das verwendete Peptid ein Polymer davon ist.

**12.** Verfahren nach Anspruch 10, worin das verwendete Peptid an einen Proteincarrier gekoppelt ist.

**13.** Verfahren nach Anspruch 12, worin das verwendete Peptid mit Humanserumalbumin konjugiert ist.

**14.** Verfahren nach Anspruch 10, worin das verwendete Peptid an ein Polymer gekoppelt ist.

**15.** Verfahren zum Herstellen eines Immunogens für die Entwicklung monoklonaler oder polyklonaler Antikörper gegen das AIDS-Virus in Säugetieren,
**dadurch gekennzeichnet,** daß
ein durch ein Verfahren nach Anspruch 1 oder Anspruch 4 hergestelltes Peptid verwendet wird.

**16.** Verfahren nach Anspruch 15, worin das verwendete Peptid ein Polymer davon ist.

**17.** Verfahren nach Anspruch 15, worin das verwendete Peptid an einen Proteincarrier gekoppelt ist.

**18.** Verfahren nach Anspruch 17, worin das Peptid mit Humanserumalbumin konjugiert ist.

**19.** Verfahren nach Anspruch 15, worin das verwendete Peptid an ein Polymer gekoppelt ist.

**20.** Verfahren zum Herstellen monoklonaler oder polyklonaler Antikörper gegen das AIDS-Virus in Säugetieren,
**dadurch gekennzeichnet,** daß
ein nach einem Verfahren gemäß einem der Ansprüche 15 bis 19 hergestelltes Immunogen verwendet wird.

**21.** Verfahren zur Herstellung eines Peptids nach Anspruch 1, worin es:

Arg-Ile-Leu-Ala-Val-Glu-Arg-Tyr-Leu-Lys-Asp-Gln-Gln-
Leu-Leu-Gly-Ile-Trp-Gly-Cys-Ser

ist.

**22.** Testkit zum Bestimmen von Antikörpern des AIDS-Virus und zur Diagnose von AIDS-, ARC- und pre-AIDS-Zuständen,
**dadurch gekennzeichnet,** daß
es

(a) einen festen Träger,
(b) ein auf einen festen Träger aufgebrachtes, eine nach einem Verfahren gemäß Anspruch 1 oder Anspruch 4 oder Anspruch 21 hergestellte Peptidzusammensetzung enthaltendes Immunoadsorbens,
(c) eine Probe eines Normalserums als Negativkontrolle,
(d) eine Probe eines Antikörpers des AIDS-Virus enthaltenden Serums als Positivkontrolle und
(e) einen Puffer zum Verdünnen der Serumproben

enthält.

## Revendications

**Revendications pour les Etats contractants suivants : BE, CH, DE, FR, GB, IT, LI, LU, NL, SE**

**1.** Composition de peptides ayant une immunoréactivité spécifique aux anticorps au virus du SIDA, caractérisée en ce qu'elle est choisie parmi des peptides contenant de douze à vingt-et-un acides aminés, dont la séquence correspond à une partie ou à la totalité de la suivante :

Arg-Ile-Leu-Ala-Val-Glu-Arg-Tyr-Leu-Lys-Asp-Gln-Gln-Leu-
Leu-Gly-Ile-Trp-Gly-Cys-Ser;

leurs analogues dans lesquels les acides aminés dans la séquence sont substitués, supprimés ou ajoutés aussi longtemps que l'immunoréactivité aux anticorps au virus du SIDA est préservée; et des conjugués de peptides ou leurs analogues.

**2.** Peptide selon la revendication 1, dans lequel un ou plusieurs acides aminés sont supprimés de l'une ou l'autre terminaison de la séquence d'acides aminés.

**3.** Peptide selon la revendication 2, qui comprend au moins quinze acides aminés.

**4.** Peptide synthétisé chimiquement in vitro, caractérisé en ce qu'il comprend une séquence d'acides aminés correspondant sensiblement à une séquence d'acides aminés antigène présente dans la protéine enveloppe du virus du SIDA, la séquence d'acides aminés prise de la gauche vers la droite et dans la direction de la N-terminaison vers la C-terminaison comprenant une des séquences d'acides aminés suivantes :
(a) -Trp-Gly-Cys-Ser-;
(b) -Asp-Gln-Gln-Leu-Leu-Gly-Ile-;
et
(c) -Ile-Leu-Ala-Val-Glu-Arg-Tyr-Leu-Lys-.

**5.** Procédé pour la détection d'anticorps au virus du SIDA et le diagnostic du SIDA, de l'ARC et des états présidéens, caractérisé en ce qu'il comprend :
(a) la préparation d'un peptide selon la revendication 1 ou 4;
et
(b) l'utilisation d'une quantité efficace du peptide comme antigène enrobant pour détecter les anticorps au virus du SIDA dans un procédé d'immunodosage.

**6.** Procédé selon la revendication 5, dans lequel le procédé d'immunodosage est un procédé de dosage en bande multipoint, ELISA, IRMA, d'hémagglutination.

**7.** Procédé selon la revendication 6, dans lequel pour chaque test, on utilise d'environ 0,1 à environ 20 µg du peptide dans un tampon à un pH d'environ 7 à environ 10 en tant qu'antigène.

**8.** Procédé selon la revendication 6, dans lequel, pour chaque test, on utilise environ 1 µg du peptide dans un tampon à un pH d'environ 7 à 10 en tant qu'antigène.

**9.** Procédé selon la revendication 7, dans lequel le tampon est un tampon de bicarbonate 10 mM à un pH d'environ 9 à environ 10.

10. Procédé selon la revendication 9, dans lequel le tampon est un tampon de bicarbonate 10 mM à un pH d'environ 9,5.

11. Procédé selon la revendication 7, dans lequel le tampon est un tampon TRIS 50 mM à un pH d'environ 7 à 8.

12. Procédé selon la revendication 11, dans lequel le tampon est un tampon TRIS 50 mM à un pH d'environ 7,5.

13. Ensemble de tests pour la détection d'anticorps au virus du SIDA et le diagnostic du SIDA, de l'ARC et des états pré-sidéens, caractérisé en ce que l'immuno-adsorbant comprend une composition de peptide selon la revendication 1 ou 4.

14. Vaccin pour provoquer la production d'anticorps contre le virus du SIDA chez les mammifères, caractérisé en ce qu'il comprend un peptide selon la revendication 1 ou la revendication 4.

15. Vaccin selon la revendication 14, dans lequel le peptide est un de ses polymères.

16. Vaccin selon la revendication 14, dans lequel le peptide est associé à un porteur protéinique.

17. Vaccin selon la revendication 16 dans lequel le peptide est conjugué à de l'albumine de sérum humain.

18. Vaccin selon la revendication 14, dans lequel le peptide est associé à un polymère.

19. Immunogène pour le développement d'anticorps monoclonaux et polyclonaux au virus du SIDA chez les mammifères, caractérisé en ce que l'on utilise un peptide selon la revendication 1 ou la revendication 4.

20. Immunogène selon la revendication 19, dans lequel le peptide est un de ses polymères.

21. Immunogène selon la revendication 19, dans lequel le peptide est associé à un porteur protéinique.

22. Immunogène selon la revendication 21 dans lequel le peptide est conjugué à de l'albumine de sérum humain.

23. Immunogène selon la revendication 19, dans lequel le peptide est associé à un polymère.

24. Procédé pour la production d'anticorps monoclonaux et polyclonaux au virus du SIDA chez les mammifères caractérisé en ce qu'il comprend un immunogène selon l'une quelconque des revendications 19 à 23.

25. Anticorps monoclonaux ou polyclonaux contre le virus du SIDA, caractérisés en ce qu'ils sont préparés par un procédé selon la revendication 24.

26. Peptide selon la revendication 1, dans lequel il est :

Arg-Ile-Leu-Ala-Val-Glu-Arg-Tyr-Leu-Lys-Asp-Gln-Gln-Leu-
Leu-Gly-Ile-Trp-Gly-Cys-Ser.

27. Ensemble de tests pour la détection d'anticorps au virus du SIDA et le diagnostic du SIDA, de l'ARC, et des états pré-sidéens caractérisé en ce qu'il comprend :
(a) un support solide;
(b) déposé sur le support solide, un immuno-adsorbant comprenant une composition de peptide selon la revendication 1 ou la revendication 4 ou la revendication 26;
(c) un échantillon d'un sérum normal comme témoin négatif

(d) un échantillon de sérum contenant des anticorps au virus du SIDA comme témoin positif; et
(e) un tampon pour diluer les échantillons de sérum.

**Revendications pour l'Etat contractant suivant : AT**

1. Procédé pour la production d'un peptide ayant une immunoréactivité aux anticorps au virus du SIDA, caractérisé en ce qu'on synthétise chimiquement un peptide choisi parmi les peptides contenant de douze à vingt-et-un acides aminés, dont la séquence correspond à une partie ou à la totalité de la suivante:

   Arg-Ile-Leu-Ala-Val-Glu-Arg-Tyr-Leu-Lys-Asp-Gln-Gln-Leu-Leu-Gly-Ile-Trp-Gly-Cys-Ser;

   leurs analogues dans lesquels les acides aminés dans la séquence sont substitués, supprimés ou ajoutés aussi longtemps que l'immunoréactivité aux anticorps au virus du SIDA est préservée; et les conjugués des peptides ou leurs analogues.

2. Procédé selon la revendication 1, dans lequel un ou plusieurs acides aminés sont supprimés de l'une ou l'autre terminaison de la séquence d'acides aminés.

3. Procédé selon la revendication 2, dans lequel le peptide produit comprend au moins quinze acides aminés.

4. Procédé pour la production d'un peptide in vitro caractérisé en ce qu'il comprend la synthèse chimique d'une séquence d'acides aminés correspondant sensiblement à une séquence d'acides aminés antigène présente dans la protéine enveloppe du virus du SIDA, la séquence d'acides aminés produite prise de la gauche vers la droite et dans la direction de la N-terminaison vers la C-terminaison comprenant une des séquences d'acides aminés suivantes :
   (a) -Trp-Gly-Cys-Ser-;
   (b) -Asp-Gln-Gln-Leu-Leu-Gly-Ile-; et
   (c) -Ile-Leu-Ala-Val-Glu-Arg-Tyr-Leu-Lys-.

5. Procédé pour la détection d'anticorps au virus du SIDA et le diagnostic du SIDA, de l'ARC et des états présidéens, caractérisé en ce qu'il comprend :
   (a) la préparation d'un peptide selon la revendication 1 ou 4;
   et
   (b) l'utilisation d'une quantité efficace du peptide comme antigène enrobant pour détecter les anticorps au virus du SIDA dans un procédé d'immunodosage.

6. Procédé selon la revendication 5, dans lequel le procédé d'immunodosage est un procédé en bande multipoint, ELISA, IRMA, d'hémagglutination.

7. Procédé selon la revendication 6, dans lequel, pour chaque test, on utilise d'environ 0,1 à environ 20 µg du peptide dans un tampon à un pH de 7 à environ 10 en tant qu'antigène.

8. Procédé selon la revendication 6, dans lequel, pour chaque test, on utilise environ 1 µg du peptide dans un tampon à un pH d'environ 7 à 10 en tant qu'antigène.

9. Ensemble de tests pour la détection d'anticorps du virus du SIDA et le diagnostic du SIDA, de l'ARC et des états pré-sidéens, caractérisé en ce que l'immuno-adsorbant comprend une composition de peptide produit selon la revendication 1 ou 4.

10. Procédé pour la production d'un vaccin pour provoquer la production d'anticorps contre le virus du SIDA chez les mammifères, caractérisé en ce qu'il comprend l'utilisation d'un peptide produit par un procédé tel que revendiqué à la revendication 1 ou à la revendication 4.

**11.** Procédé selon la revendication 10, dans lequel le peptide utilisé est un de ses polymères.

**12.** Procédé selon la revendication 10, dans lequel le peptide est associé à un porteur protéinique.

**13.** Procédé selon la revendication 12 dans lequel le peptide est conjugué à de l'albumine de sérum humain.

**14.** Procédé selon la revendication 10, dans lequel le peptide utilisé est associé à un polymère.

**15.** Procédé pour la production d'un immunogène pour le développement d'anticorps monoclonaux et polyclonaux au virus du SIDA chez les mammifères caractérisé en ce que l'on utilise un peptide selon la revendication 1 ou la revendication 4.

**16.** Procédé selon la revendication 15, dans lequel le peptide utilisé est un de ses polymères.

**17.** Procédé selon la revendication 15, dans lequel le peptide utilisé est associé à un porteur protéinique.

**18.** Procédé selon la revendication 17 dans lequel le peptide est conjugué à de l'albumine de sérum humain.

**19.** Procédé selon la revendication 15, dans lequel le peptide utilisé est associé à un polymère

**20.** Procédé pour la production d'anticorps monoclonaux et polyclonaux au virus du SIDA chez les mammifères caractérisé en ce qu'il comprend un immunogène produit selon l'une quelconque des revendications 15 à 19.

**21.** Procédé pour la production d'un peptide selon la revendication 1, dans lequel il est :

Arg-Ile-Leu-Ala-Val-Glu-Arg-Tyr-Leu-Lys-Asp-Gln-Gln-Leu-
Leu-Gly-Ile-Trp-Gly-Cys-Ser.

**22.** Ensemble de tests pour la détection d'anticorps au virus du SIDA et le diagnostic du SIDA, de l'ARC, et des états pré-sidéens caractérisé en ce qu'il comprend :

(a) un support solide;
(b) déposé sur le support solide, un immuno-adsorbant comprenant une composition de peptide produite par un procédé tel que revendiqué à la revendication 1 ou la revendication 4 ou à la revendication 21;
(c) un échantillon d'un sérum normal comme témoin négatif;
(d) un échantillon de sérum contenant des anticorps au virus du SIDA comme témoin positif; et
(e) un tampon pour diluer les échantillons de sérum.